# EUROPEAN PATENT APPLICATION

(11) **EP 4 600 419 A1**
(43) Date of publication of application: **13.08.2025**
(21) Application number: 22962159.4
(22) Date of filing: 13.10.2022
(51) Int. Cl.: D06N 3/00, D06N 3/14, D06M 16/00, D06M 15/564, D06M 15/03, D06M 15/17, C12N 1/20

(54) **METHOD FOR MANUFACTURING VEGETABLE TANNED LEATHER USING MUSHROOM MYCELIUM**

(71) Applicant: Agricultural Corporation, Maikoworld Co., Ltd, Jinju-si, Gyeongsangnam-do 52852 (KR)
(72) Inventor: KIM, Eun Young, Jinju-si, Gyeongsangnam-do 52849 (KR)
(74) Representative: RGTH
(86) International application number: PCT/KR2022/015510
(87) International publication number: WO 2024/080409

(57) **Abstract**

The present invention relates to a method for manufacturing vegetable tanned leather using mushroom mycelium, the method being characterized by comprising: a first step of preparing a net structure fabric; a second step of impregnating the fabric into a mixed solution; a third step of removing the mixed solution and sterilizing and cooling the fabric; a fourth step of inoculating the fabric with mushrooms; a fifth step of cultivating the mushrooms on the fabric; a sixth step of drying and pressing the fabric to produce same at a desired height; and a seventh step of mixing a resin and mushroom mycelium powder with the fabric and coating same.

## Description

### Technical Field

The present invention relates to a method for manufacturing vegetable tanned leather using mushroom mycelium, more specifically to vegetable tanned leather that is vegan leather and decomposes easily.

### Background Art

Vegans who do not use any animal products such as leather, as a new consumer class, appear to improve the welfare of animals. Animal leather and artificial leather do not decompose naturally, and therefore, they are burned or buried as waste.

### Disclosure of the Invention

### Technical Problems

Accordingly, the present invention has been made in view of the above-mentioned problems occurring in the related art, and it is an object of the present invention to provide a method for manufacturing vegetable tanned leather using mushroom mycelium that is capable of allowing the vegetable tanned leather to decompose easily.

### Technical Solutions

To accomplish the above-mentioned objects, according to the present invention, there is provided a method for manufacturing vegetable tanned leather using mushroom mycelium, the method including: a first step of preparing a net structure fabric; a second step of impregnating the fabric into a mixed solution; a third step of removing the mixed solution and sterilizing and cooling the fabric; a fourth step of inoculating the fabric with mushrooms; a fifth step of cultivating the mushrooms on the fabric; a sixth step of drying and pressing the fabric to a desired height; and a seventh step of mixing a resin and mushroom mycelium powder and coating the fabric with the mixed resin and mushroom mycelium powder.

A gap between the adjacent wound portions of the fabric may be in the range between 1 and 1. 5 cm.

In the second step, the mixed solution may be made by mixing 50 g of sugar and 5 g of soybean meal per 1 L of water, the fabric may be impregnated into the mixed solution with a temperature in the range between 15 and 25°C for 48 hours, and the sugars may be any one or more selected from sugar, glucose, and fructose.

In the third step, the fabric may be sterilized at a temperature in the range between 120 and 125°C for 10 to 20 minutes and then cooled to a temperature of 20°C.

In the fifth step, the mushrooms may be cultivated on the fabric at a temperature in the range between 18 and 25°C.

### Advantageous Effects of the Invention

According to the present invention, the method for manufacturing vegetable tanned leather using mushroom mycelium has the following advantages.

The method for manufacturing vegetable tanned leather using mushroom mycelium can cultivate the mushrooms into the pores of the fabric and coat the fabric with the resin, thereby achieving excellent productivity.

Further, the method for manufacturing vegetable tanned leather using mushroom mycelium can produce the vegetable tanned leather with the patterns of the mushrooms.

### Brief Description of Drawings

FIG. 1 is a top view showing examples of a first step in a method for manufacturing vegetable tanned leather using mushroom mycelium according to the present invention.
FIG. 2 is a flowchart showing the method for manufacturing vegetable tanned leather using mushroom mycelium according to the present invention.

### <Explanations of Reference Numerals>

S1: Step of preparing fabric
S2: Step of impregnating fabric into mixed solution
S3: Step of sterilizing and cooling fabric
S4: Step of inoculating fabric with mushrooms

### Best Mode for Invention

Hereinafter, an explanation of a method for manufacturing vegetable tanned leather using mushroom mycelium according to the present invention will be given in detail with reference to the accompanying drawings.

The method for manufacturing vegetable tanned leather using mushroom mycelium according to the present invention includes: a first step S1 of preparing a net structure fabric; a second step S2 of impregnating the fabric into a mixed solution; a third step S3 of removing the mixed solution and sterilizing and cooling the fabric; a fourth step S4 of inoculating the fabric with mushrooms; a fifth step S5 of cultivating the mushrooms on the fabric; a sixth step S6 of drying and pressing the fabric to a desired height; and a seventh step S7 of mixing a resin and mushroom mycelium powder and coating the fabric with the mixed resin and mushroom mycelium powder.

First, in the first step S1, a fabric is prepared. The fabric has a net structure. In detail, the fabric is made of natural materials such as cotton, linen, and cellulose, synthetic materials, or a mixture of natural materials and synthetic materials, but according to the present invention, the fabric may not be limited thereto.

In this case, the fabric has the net structure, and spaces are formed inside the wound fabric. In the spaces, mushrooms as will be discussed later are cultivated.

Desirably, a gap between the adjacent portions of the wound fabric is in the range between 1 and 1.5 cm. If the gap is less than 1 cm, the spaces where the mushrooms grow are not sufficient, whereas if the gap is over 1.5 cm, a volume occupied by the fabric is bulky, thereby lowering the efficiency.

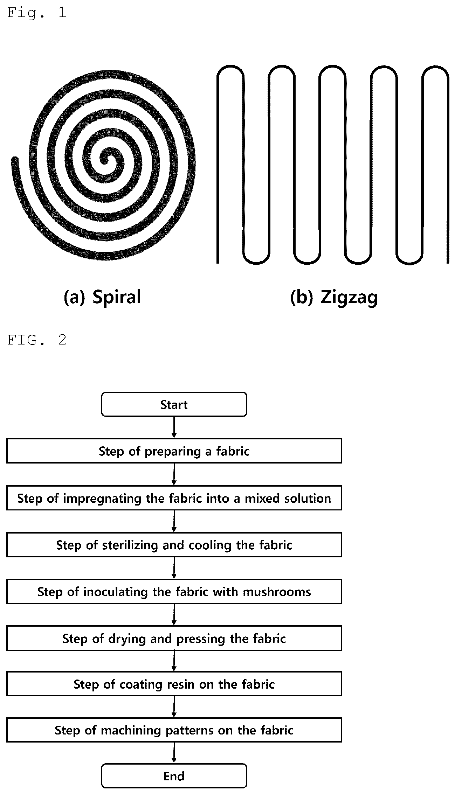

In detail, the fabric is fixedly prepared by a bar or chain in such a way as to have the gap of 1 to 1.5 cm between the adjacent wound portions thereof. As shown in FIG. 1, the fabric is prepared in a spiral or zigzag form, but it may not be limited thereto.

In the second step S2, the fabric is impregnated into a mixed solution. In detail, the fabric is submergedly impregnated into the mixed solution.

In this case, the mixed solution is made by mixing 50 g of sugar and 5 g of soybean meal per 1 L of water.

The sugar is any one or more selected from sucrose, glucose, and fructose. If an amount of the sugar is less than 50g, it is hard to cultivate the mushrooms, and contrarily, if an amount of the sugar is over 50g, the growth of the mushrooms does not increase to a great extent anymore, thereby lowering the efficiency.

If an amount of the soybean meal is less than 5g, it is hard to cultivate the mushrooms, and contrarily, if an amount of the soybean meal is over 5g, the growth of the mushrooms does not increase to a great extent anymore, thereby lowering the efficiency.

Next, the fabric is impregnated into the mixed solution with a temperature in the range between 15 and 25°C for 48 hours. If a temperature of the mixed solution is less than 15°C, it is hard to cultivate the mushrooms, and contrarily, if a temperature of the mixed solution is over 25°C, the mixed solution may be deteriorated.

If the impregnation time of the fabric is less than 48 hours, it is hard that the mixed solution is sufficiently absorbed into the fabric, and contrarily, if the impregnation time of the fabric is over 48 hours, the mixed solution is completely absorbed into the fabric, thereby lowering the efficiency.

In the third step S3, the mixed solution is removed from the fabric, and next, the fabric is sterilized and cooled. In detail, the fabric is sterilized at a temperature in the range between 120 and 125°C for 10 to 20 minutes and then cooled to a temperature of 20°C. This is to prevent the mushrooms from being contaminated or infected with bacteria.

In this case, if the sterilization temperature of the fabric is less than 120°C, the fabric is not sufficiently sterilized so that it is still contaminated, and contrarily, if the sterilization temperature of the fabric is over 125°C, the fabric is completely sterilized, thereby lowering the efficiency.

If the sterilization time of the fabric is less than 10 minutes, the fabric is not sufficiently sterilized so that it is still contaminated, and contrarily, if the sterilization time of the fabric is over 20 minutes, the fabric is completely sterilized, thereby lowering the efficiency.

In the fourth step S4, the fabric is inoculated with the mushrooms. Reishi mushrooms are used as the mushrooms, but without being limited thereto, different mushrooms may be mixedly used as the mushrooms.

In the fifth step S5, the mushrooms are cultivated on the fabric. In detail, the mushrooms are cultivated on the fabric at a temperature in the range between 18 and 25°C.

If the mushrooms are cultivated on the fabric at a temperature less than 18°C, it is hard to cultivate the mushrooms, and contrarily, if the mushrooms are cultivated on the fabric at a temperature over 25°C, the mushrooms may be deteriorated.

In the sixth step S6, the fabric is dried and pressed to a desired thickness. In detail, the fabric is completely dried, and next, the fabric moves between two rollers and is then pressed against the rollers to a desired thickness.

In the seventh step S7, a resin is coated onto the fabric. In detail, the resin is coated onto the surface of the fabric. In this case, the resin is mixed with mushroom mycelium powder. Reishi mushroom mycelium powder is used as the mushroom mycelium powder, but without being limited thereto, different types of mushroom mycelia may be mixedly used.

The patterns of the mushrooms may be used as the patterns of the fabric, but of course, a step of machining patterns on the fabric may be additionally performed.

### Mode for Invention

Hereinafter, explanations of tests for the method for manufacturing vegetable tanned leather using mushroom mycelium according to the present invention will be given in detail.

### 1. Tests for spirally wound fabric with given gap

Tensile strength and elongation for pieces of vegetable tanned leather with the same size as one another, which are manufactured according to the following embodiments, were measured according to ASTM test methods, and degrees of shape stability for them were evaluated by an evaluation team consisting of five artificial leather professionals that consulted the results of their tensile strength and elongation and performed sensory methods.

### [Embodiment 1]

According to an embodiment 1, a net structure cotton fabric was spirally wound with a gap of 0.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 2]

According to an embodiment 2, a net structure cotton fabric was spirally wound with a gap of 1 cm and completely submerged into a mixed solution in which 50 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 3]

According to an embodiment 3, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 4]

According to an embodiment 4, a net structure cotton fabric was spirally wound with a gap of 2 cm and completely submerged into a mixed solution in which 50 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

**[Table 1]**

| Classification | Embodiment 1 | Embodiment 2 | Embodiment 3 | Embodiment 4 |
|---|---|---|---|---|
| Fabric gap | 0.5 cm | 1 cm | 1.5 cm | 2 cm |
| Weight | 453 g | 468 g | 505 g | 512 g |
| Tensile strength (longitudinal) | 21. 4 kg/inch | 27.2 kg/inch | 41.1 kg/inch | 42.0 kg/inch |
| Tensile strength (transverse) | 25.1 kg/inch | 30.5 kg/inch | 42.8 kg/inch | 43.4 kg/inch |
| Elongation (longitudinal) | 50.8% | 43.2% | 31.5% | 30.1% |
| Elongation (transverse) | 55.1% | 48.1% | 33.1% | 32.9% |
| Shape stability | Poor | Good | Very good | Very good |

As appreciated from Table 1, it can be checked that the embodiments 2 to 4 have excellent characteristics, and in this case, the embodiment 3 has the best efficiency, high tensile strength, and longitudinal and transverse elongation ratios with no big difference, so that the shape stability is very good.

### 2. Tests for ratios of mixed solution

Tensile strength and elongation for pieces of vegetable tanned leather with the same size as one another, which are manufactured according to the following embodiments, were measured according to ASTM test methods, and degrees of shape stability for them were evaluated by an evaluation team consisting of five artificial leather professionals that consulted the results of their tensile strength and elongation and performed sensory methods.

### [Embodiment 5]

According to an embodiment 5, a net structure cotton fabric was spirally wound with a gap of 1.5 cm and completely submerged into a mixed solution in which 50 g of sugar and 1 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 6]

According to an embodiment 6, a net structure cotton fabric was spirally wound with a gap of 1.5 cm and completely submerged into a mixed solution in which 50 g of sugar and 3 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 7]

According to an embodiment 7, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 8]

According to an embodiment 8, a net structure cotton fabric was spirally wound with a gap of 1.5 cm and completely submerged into a mixed solution in which 50 g of sugar and 8 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 9]

According to an embodiment 9, a net structure cotton fabric was spirally wound with a gap of 1.5 cm and completely submerged into a mixed solution in which 50 g of sugar and 10 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 10]

According to an embodiment 10, a net structure cotton fabric was spirally wound with a gap of 1.5 cm and completely submerged into a mixed solution in which 40 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 11]

According to an embodiment 11, a net structure cotton fabric was spirally wound with a gap of 1.5 cm and completely submerged into a mixed solution in which 45 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 12]

According to an embodiment 12, a net structure cotton fabric was spirally wound with a gap of 1.5 cm and completely submerged into a mixed solution in which 55 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 13]

According to an embodiment 13, a net structure cotton fabric was spirally wound with a gap of 1.5 cm and completely submerged into a mixed solution in which 60 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

**[Table 2]**

| Classification | Embodiment 5 | Embodiment 6 | Embodiment 7 | Embodiment 8 | Embodiment 9 |
|---|---|---|---|---|---|
| Water | 1 L | 1 L | 1 L | 1 L | 1 L |
| Sugar | 50 g | 50 g | 50 g | 50 g | 50 g |
| Soybean meal | 1 g | 3 g | 5 g | 8 g | 10 g |
| Weight | 470 g | 483 g | 505 g | 511 g | 515 g |
| Tensile strength (longitudinal) | 27.1 kg/inch | 32.2 kg/inch | 41. 1 kg/inch | 42.9 kg/inch | 43.2 kg/inch |
| Tensile strength (transverse) | 30.5 kg/inch | 35.1 kg/inch | 42.8 kg/inch | 43.5 kg/inch | 45.1 kg/inch |
| Elongation (longitudinal) | 40.8% | 38.5% | 31.5% | 30.6% | 30.1% |
| Elongation (transverse) | 42.1% | 39.9% | 33.1% | 32.8% | 31.0% |
| Shape stability | Poor | Good | Very good | Very good | Very good |

| Classification | Embodiment 10 | Embodiment 11 | Embodiment 12 | Embodiment 13 | |
|---|---|---|---|---|---|
| Water | 1 L | 1 L | 1 L | 1 L | |
| Sugar | 40 g | 45 g | 55 g | 60 g | |
| Soybean meal | 5 g | 5 g | 5 g | 5 g | |
| Weight | 430 g | 463 g | 516 g | 520 g | |
| Tensile strength (longitudinal) | 23.2 kg/inch | 30.1 kg/inch | 43.1 kg/inch | 43.9 kg/inch | |
| Tensile strength (transverse) | 25.8 kg/inch | 32.5 kg/inch | 44.8 kg/inch | 45.1 kg/inch | |
| Elongation (longitudinal) | 48.1% | 40.9% | 30.0% | 29.5% | |
| Elongation (transverse) | 49.5% | 43.4% | 32.1% | 31.5% | |
| Shape stability | Poor | Good | Very good | Very good | |

As appreciated from Table 2, it can be checked that as the weights of the sugar and soybean meal increase, tensile strength, elongation, and shape stability become excellent, but if their weight increase more than those in the sixth embodiment, it can be checked that the efficiencies do not significantly increase.

### 3. Tests for impregnation conditions

Tensile strength and elongation for pieces of vegetable tanned leather with the same size as one another, which are manufactured according to the following embodiments, were measured according to ASTM test methods, and degrees of shape stability for them were evaluated by an evaluation team consisting of five artificial leather professionals that consulted the results of their tensile strength and elongation and performed sensory methods.

### [Embodiment 14]

According to an embodiment 14, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 10°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 15]

According to an embodiment 15, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 15°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 16]

According to an embodiment 16, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 17]

According to an embodiment 17, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 25°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 18]

According to an embodiment 18, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 30°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 19]

According to an embodiment 19, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 43 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 20]

According to an embodiment 20, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 45 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 21]

According to an embodiment 21, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 50 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 22]

According to an embodiment 22, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 53 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

**[Table 3]**

| Classification | Embodiment 14 | Embodiment 15 | Embodiment 16 | Embodiment 17 | Embodiment 18 |
|---|---|---|---|---|---|
| Impregnation temperature | 10°C | 15°C | 20°C | 25°C | 30°C |
| Impregnation time | 48 hours | 48 hours | 48 hours | 48 hours | 48 hours |
| Weight | 460 g | 477 g | 505 g | 495 g | 450 g |
| Tensile strength (longitudinal) | 28.1 kg/inch | 32.8 kg/inch | 41.1 kg/inch | 39.1 kg/inch | 25.5 kg/inch |
| Tensile strength (transverse) | 31.5 kg/inch | 34.1 kg/inch | 42.8 kg/inch | 40.9 kg/inch | 27.1 kg/inch |
| Elongation (longitudinal) | 43.8 | 39.6% | 31.5% | 32.8% | 50.8% |
| Elongation (transverse) | 45.2% | 40.5% | 33.1% | 35.1% | 53.2% |
| Shape stability | Poor | Good | Very good | Good | Poor |

| Classification | Embodiment 19 | Embodiment 20 | Embodiment 21 | Embodiment 22 | |
|---|---|---|---|---|---|
| Impregnation temperature | 20°C | 20°C | 20°C | 20°C | |
| Impregnation time | 43 hours | 45 hours | 50 hours | 53 hours | |
| Weight | 470 g | 483 g | 513 g | 515 g | |
| Tensile strength (longitudinal) | 29.8 kg/inch | 31.5 kg/inch | 42.8 kg/inch | 43.3 kg/inch | |
| Tensile strength (transverse) | 31.8 kg/inch | 33.9 kg/inch | 44.2 kg/inch | 45.1 kg/inch | |
| Elongation (longitudinal) | 43.1% | 40.5% | 30.8% | 30.2% | |
| Elongation (transverse) | 45.1% | 42.1% | 32.1% | 32.0% | |
| Shape stability | Poor | Good | Very good | Very good | |

As appreciated from Table 3, it can be checked that if the fabric is impregnated into the mixed solution with the temperature over 25°C, the mixed solution becomes deteriorated to cause the shape stability to be lowered, and if the impregnation time of the fabric is over 48 hours, it can be checked that the efficiencies do not significantly increase.

### 4. Tests for cultivation conditions

Tensile strength and elongation for pieces of vegetable tanned leather with the same size as one another, which are manufactured according to the following embodiments, were measured according to ASTM test methods, and degrees of shape stability for them were evaluated by an evaluation team consisting of five artificial leather professionals that consulted the results of their tensile strength and elongation and performed sensory methods.

### [Embodiment 23]

According to an embodiment 23, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 15°C for 20 days.

The fabric was completely dried and pressed to a thickness of 1 mm. Next, polyurethane and mushroom mycelium powder were coated onto the surface of the fabric to make the vegetable tanned leather.

### [Embodiment 24]

According to an embodiment 24, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 20°C for 20 days.

The fabric was completely dried and pressed to a thickness of 1 mm. Next, polyurethane and mushroom mycelium powder were coated onto the surface of the fabric to make the vegetable tanned leather.

### [Embodiment 25]

According to an embodiment 25, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 25°C for 20 days.

The fabric was completely dried and pressed to a thickness of 1 mm. Next, polyurethane and mushroom mycelium powder were coated onto the surface of the fabric to make the vegetable tanned leather.

### [Embodiment 26]

According to an embodiment 26, a net structure cotton fabric was spirally wound with a gap of 1.5 cm and completely submerged into a mixed solution in which 50 g of sugar and 5 g of soybean meal per 1 L of water were mixed. In this case, the mixed solution had a temperature of 20°C, and the fabric was impregnated into the mixed solution for 48 hours. Next, the fabric was sterilized at a temperature of 121°C for 15 minutes and then cooled to a temperature of 20°C, and the fabric was inoculated with reishi mushrooms to allow the reishi mushrooms to be cultivated on the fabric at a temperature of 30°C for 20 days.

The fabric was completely dried and pressed to a thickness of 1 mm. Next, polyurethane and mushroom mycelium powder were coated onto the surface of the fabric to make the vegetable tanned leather.

**[Table 4]**

| Classification | Embodiment 23 | Embodiment 24 | Embodiment 25 | Embodiment 26 |
|---|---|---|---|---|
| Cultivation temperature | 15°C | 20°C | 25°C | 30°C |
| Cultivation time | 20 days | 20 days | 20 days | 20 days |
| Weight | 467 g | 505 g | 508 g | 491 g |
| Tensile strength (longitudinal) | 27.8 kg/inch | 41.1 kg/inch | 41.5 kg/inch | 38.2 kg/inch |
| Tensile strength (transverse) | 30.1 kg/inch | 42.8 kg/inch | 43.3 kg/inch | 41.6 kg/inch |
| Elongation (longitudinal) | 43.8% | 31.5% | 30.8% | 38.1% |
| Elongation (transverse) | 45.2% | 33.1% | 32.5% | 40.2% |
| Shape stability | Good | Very good | Good | Poor |

As appreciated from Table 4, it can be checked that if the cultivation temperature is over 25°C, the mushrooms become deteriorated to cause the shape stability to be lowered.

The disclosed embodiments are merely exemplary of the invention, which can be embodied in various forms. Therefore, specific structural and functional details disclosed herein are not to be interpreted as limiting, but merely as a basis for the claims and as a representative basis for teaching one of ordinary skill in the art to variously employ the present invention in virtually any appropriately detailed structure.

Although specific embodiments have been illustrated and described herein, it will be appreciated by those of ordinary skill in the art that any arrangement, which is calculated to achieve the same purpose, may be substituted for the specific embodiment shown. This application is intended to cover any adaptations or variations of the present invention. Therefore, it is manifestly intended that this invention be limited only by the claims and the equivalents thereof.

## Claims

1. A method for manufacturing vegetable tanned leather using mushroom mycelium, the method comprising:
a first step of preparing a net structure fabric;
a second step of impregnating the fabric into a mixed solution;
a third step of removing the mixed solution and sterilizing and cooling the fabric;
a fourth step of inoculating the fabric with mushrooms;
a fifth step of cultivating the mushrooms on the fabric;
a sixth step of drying and pressing the fabric to a desired height; and
a seventh step of mixing a resin and mushroom mycelium powder and coating the fabric with the mixed resin and mushroom mycelium powder.

2. The method according to claim 1, wherein in the first step, a gap between the adjacent wound portions of the fabric is in the range between 1 and 1. 5 cm.

3. The method according to claim 1, wherein in the second step, the mixed solution is made by mixing 50 g of sugar and 5 g of soybean meal per 1 L of water, the fabric is impregnated into the mixed solution with a temperature in the range between 15 and 25°C for 48 hours, and the sugar is any one or more selected from sucrose, glucose, and fructose.

4. The method according to claim 1, wherein in the third step, the fabric is sterilized at a temperature in the range between 120 and 125°C for 10 to 20 minutes and then cooled to a temperature of 20°C.

5. The method according to claim 1, wherein in the fifth step, the mushrooms are cultivated on the fabric at a temperature in the range between 18 and 25°C.
